# EUROPÄISCHE PATENTSCHRIFT

(11) **EP 2 083 732 B1**
(45) Veröffentlichungstag und Bekanntmachung des Hinweises auf die Patenterteilung: **30.12.2015**
(21) Anmeldenummer: 07820933.5
(22) Anmeldetag: 04.10.2007
(51) Int. Cl.: A61B 18/14

(54) **BIEGEWEICHER KATHETER ZUR HOCHFREQUENZTHERAPIE VON BIOLOGISCHEM GEWEBE**
FLEXIBLE CATHETER FOR THE HIGH-FREQUENCY THERAPY OF BIOLOGICAL TISSUE
CATHÉTER FLEXIBLE POUR UNE THÉRAPIE HAUTE FRÉQUENCE SUR UN TISSU

(30) Priorität: 04.10.2006 DE 102006047366
(43) Veröffentlichungstag der Anmeldung: 05.08.2009
(73) Patentinhaber: OLYMPUS Winter & Ibe GmbH, 22045 Hamburg (DE)
(72) Erfinder: FAY, Markus, 14513 Teltow (DE); KÜHNE, Wolfgang, 16567 Schönfliess (DE)
(74) Vertreter: Eisenführ Speiser
(86) Internationale Anmeldenummer: PCT/EP2007/060556
(87) Internationale Veröffentlichungsnummer: WO 2008/040788

(56) Entgegenhaltungen:
- WO-A-96/37146
- US-A- 5 423 878
- US-A1- 2004 116 793

## Beschreibung

Die Erfindung betrifft einen biegeweichen Katheter aus vorzugsweise biokompatiblem Kunststoff mit zumindest einer am distalen Ende des Katheters angebrachten Elektrode zur Hochfrequenztherapie von biologischem Gewebe.

Katheter zur Hochfrequenztherapie sind grundsätzlich bekannt. Während einer Hochfrequenztherapie wird durch Anlegen einer hochfrequenten Wechselspannung zwischen zwei Elektroden ein elektrischer Strom in dem zwischen den Elektroden liegendem Körpergewebe hervorgerufen, der eine Erwärmung des die Elektroden umgebenden Körpergewebes zur Folge hat. Bei einem Kathetertyp gemäß dieser Anmeldung stehen die Elektroden nach dem Einführen in ein Körperlumen (z.B. Blutgefäß oder Gallengang) des Patienten mit dem Körpergewebe in elektrisch leitfähiger Verbindung. Durch den ohmschen Widerstand des Körpergewebes erfolgt eine Umsetzung des über die Elektroden applizierten Wechselstroms in joulesche Wärme. Bei Temperaturen zwischen 50°C und 100°C kommt es zu einer Denaturierung der körpereigenen Proteine (Koagulation) und in der Folge zum Schrumpfen bzw. Absterben der betroffenen Gewebsareale. Auf Grund der hohen Stromdichte an den aktiven Elektroden erfolgt die Erwärmung vorwiegend im Bereich dieser Elektroden, so dass ein lokal begrenzter thermischer Einsatz möglich ist.

Aus US 5,782,760 und WO 2006/017754 A1 sind Katheter zur Hochfrequenztherapie von Hohlorganen bekannt.

Katheter zur Hochfrequenztherapie von oder in Hohlorganen der gattungsgemäßen Art umfassen
- ein biegeweiches Schaftrohr aus biokompatiblem Kunststoff, dessen innere Mantelfläche ein Lumen umschließt, und dessen äußere Mantelfläche den Außenmantel des Katheterschafts darstellt,
- einen distalen Schaftabschnitt mit wenigstens einer Elektrode zur Abgabe hochfrequenten Wechselstroms an das den Katheter im Behandlungsfall umgebende Gewebe,
- eine mit der Elektrode elektrisch leitend verbundene elektrische Leitung, die bis zum proximalen Ende des Katheters reicht,
- ein am distalen Ende des biegeweichen Schaftrohres befestigtes distales Endstück.

Ein Katheter nach der Präambel von Anspruch 1 ist aus US 542 3878 bekannt.

Der vorliegenden Erfindung liegt nun die Aufgabe zugrunde, einen Katheter zur Hochfrequenztherapie in zum Teil flüssigkeitsgefüllten Körperlumen bereitzustellen, der eine zuverlässige Funktion und einen einfachen Aufbau bietet.

Erfindungsgemäß wird diese Aufgabe durch einen Katheter nach Anspruch 1 gelöst, der über ein distales Endstück verfügt, das mit einem proximalen Endabschnitt in das Lumen des biegeweichen Schaftrohres hineinragt und dort eine Klemmverbindung mit dem distalen Ende des Schaftrohres bildet. Die Klemmverbindung wird hierbei durch eine Hülse in Form eines Hohlzylinders gesichert, welche eine radial wirkende Klemmkraft auf die Klemmverbindung ausübt. Vorzugsweise ist die Hülse dafür auf die äußere Mantelfläche des biegeweichen Schaftrohres aufgezogen, um die Klemmverbindung durch eine radial nach innen wirkende Kraft von außen zu sichern. Für den Fall, dass das Endstück eine zentrale Durchgangsbohrung besitzt, kann die Hülse gemäß einer weiteren Ausführungsvariante auch in das Endstück eingeschoben werden und so die Klemmverbindung durch eine radial nach außen wirkende Kraft von Innen sichern.

Ist die Hülse auf das biegeweiche Schaftrohr aufgezogen, kann sie gemäß einer besonders bevorzugten Ausführungsform als Elektrode ausgeführt sein.

Bevorzugt besitzt das distale Endstück einen proximalen Abschnitt mit zumindest einer Querschnittsveränderung auf der äußeren Mantelfläche, die ein Verschieben des Endstücks in axialer Richtung erschwert. Die Querschnittsveränderung hat vorzugsweise die Form eines radial nach außen stehenden Vorsprungs.

Der Katheterschaft ist entsprechend seiner zwei Hauptausführungsformen entweder am distalen Ende verschlossen oder verfügt über eine Öffnung am distalen Ende des Schafts. In dieser als zweites genannten Hauptausführungsform verfügt der Katheterschaft über ein inneres - vom distalen zum proximalen Ende des Katheters durchgehendes - Lumen, welches bevorzugt so ausgebildet ist, dass ein Führungsdraht durch die proximale Öffnung in das Lumen eindringen und durch die distale Öffnung wieder austreten kann. Ist der Katheter gemäß der als erstes genannten Hauptausführungsform am distalen Ende verschlossen, kann er entsprechend einer weiteren Ausgestaltung über ein Kühlsystem verfügen, das mit einem proximalen Zu- und Abfluss eines Kühlmittels verbunden ist und welches innerhalb des zum distalen Ende verschlossenen Lumens des Katheters zirkuliert.

Die Elektrode besteht bevorzugt aus einem leitenden, biokompatiblen Material. In einer besonders bevorzugten Ausführungsvariante besteht zumindest die distale Elektrode ganz oder in Teilen aus chirurgischem Edelstahl. Sie kann auch gemäß weiteren Ausgestaltungen aus Platin, Titan, Iridium oder Gold gefertigt sein. In einer bevorzugten Ausführungsvariante hat zumindest die distale Elektrode eine Wandstärke von 0,1 mm und weist entlang der Achse des Katheters eine Ausdehnung von in etwa 5,4 mm auf. In einer weiteren bevorzugten Ausführungsform besitzt der Katheter zwei Ringelektroden, von denen die proximale und die distale Elektrode in den Abmessungen einander gleichen.

In beiden Hauptvarianten ist bevorzugt eine weitere Elektrode als proximale Elektrode auf das Schaftrohr aufgezogen. Diese kann aus gleichem Material bestehen und gleich aufgebaut sein, wie die distale Elektrode. Möglich ist aber auch eine flexiblere Ausgestaltung der Elektrode aus zum Beispiel geflochtenem Draht, manschettenartigen Segmenten, in Form einer Helixwendel oder ähnlichem, um die Biegsamkeit des gesamten Katheterschaftes zu gewährleisten, bzw. im Vergleich zu einem Schaftrohr mit mehreren starren Elektroden zu erhöhen.

Gemäß weiteren Ausführungsvarianten sind drei oder mehr Elektroden auf das Schaftrohr aufgezogen. Bei jeder bi- oder multipolaren Ausgestaltung des Katheters ist zwischen den Elektroden bevorzugt ein Isolator auf das Schaftrohr aufgezogen, der in einer bevorzugten Ausführungsvariante eine Wandstärke von ebenfalls 0,1 mm besitzt und entlang der Achse des Katheterschafts eine Ausdehnung von ca. 10% der axialen aktiven Länge der Elektroden aufweist. Vorzugsweise ist dieser Isolator aus PEEK geformt.

Die Elektroden sind über Leitungen mit einem am proximalen Ende des Katheterschafts angeordneten Anschluss für einen Hochfrequenzgenerator elektrisch leitend verbunden. In einer bevorzugten Ausführungsform bestehen die Leitungen, die bevorzugt in dem Mantel des Schaftrohres eingebettet verlaufen, aus Kupfer und haben einen Durchmesser von bevorzugt 0,15 mm.

Verlaufen die Leitungen gemäß einer weiteren Ausführungsform nicht eingebettet in dem Mantel, sondern entlang der inneren Mantelfläche des Schaftrohres, ist es besonders in der Ausführungsvariante des Katheters mit offenem distalen Ende notwendig, die Leitungen gegenüber der eindringenden Flüssigkeit zu isolieren. Ist der Katheter dafür vorgesehen, auf einen Führungsdraht aufgezogen zu werden, ist es darüber hinaus notwendig, die Leitungen gegenüber mechanischen Belastungen durch den Führungsdraht zu schützen. Insbesondere für diese beiden Fälle ist gemäß einer weiteren Ausführungsform vorgesehen, in das Lumen des flexiblen Schaftrohres einen Kunststoffschlauch einzuziehen.

Gemäß dieser Ausgestaltungsvariante verlaufen die Leitungen zwischen der äußeren Mantelfläche des Schlauches und der inneren Mantelfläche des Schaftrohres. Bevorzugt sind der Außendurchmesser des Kunststoffschlauches und der Innendurchmesser des Schaftrohres so gewählt, dass nach dem Einziehen des Kunsstoffschlauchs in das Lumen des Schaftrohres, dieser flüssigkeitsdicht mit dem Mantel des Schaftrohres verbunden ist, so dass in den Zwischenraum zwischen Schaftrohr und Kunststoffschlauch keine Flüssigkeit eindringen kann. Eine flüssigkeitsdichte Anordnung kann auch durch verkleben oder verschweißen erreicht werden. Um einen möglichen gegenseitigen elektrischen Kontakt der Leitungen und damit einen Kurzschluss zu vermeiden, können die Leitungen zwischen Elektrode und proximalem Ende des Katheters auch einzeln mit einem elektrisch isolierenden Material überzogen sein. Die Verbindung von Kunststoffschlauch und Schaftrohr muss dann nicht notwendigerweise flüssigkeitsdicht ausgeführt sein.

Am distalen Ende des Schaftrohres sind die Leitungen mit den Elektroden vorzugsweise durch eine Klemmkontaktierung verbunden. Hierbei werden die Leitungen durch die radial nach innen wirkende Klemmkraft der Elektroden zwischen Elektrode und Schaftrohr eingeklemmten, so dass ein elektrischer Kontakt zustande kommt. Um die elektrischen Leitungen von dem Zwischenraum zwischen Mantelinnenfläche des Schaftrohres und der Mantelaußenfläche des Kunststoffschlauches durch den Mantel des Schaftrohres auf die Mantelaußenfläche des Schaftrohres zu führen, sind in der Mantelfläche des Schaftrohres radial ausgerichtete Durchführungen vorgesehen. Durch diese Durchführungen sind die distalen Endabschnitte der Leitungen hindurchgeführt und so gebogen, dass sie in Längsrichtung des Schaftrohres zwischen der äußeren Mantelfläche des Schaftrohres und der jeweiligen Elektrode verlaufen und dort eingeklemmt sind.

Der Kunststoffschlauch, der in das Lumen des Schaftrohres eingezogen ist, ist aus einem biokompatiblen Polymer, und zwar vorzugsweise aus Polyimid (PI) gefertigt. Er weist vorzugsweise einen Innendurchmesser auf, der dafür geeignet ist, einen Führungsdraht mit einem Außendurchmesser von 0,025 Inch oder 0,035 Inch aufzunehmen. In einer bevorzugten Ausführungsform hat der Kunststoffschlauch einen Innendurchmesser von 0,65 bis 1,0 mm, in einer besonders bevorzugten Ausführungsform einen Innendurchmesser von 0,81 mm. Die innere Mantelfläche des Kunststoffschlauches kann mit einer Anti-Haft-Beschichtung versehen sein. Entsprechend kann, gemäß der weiter oben beschriebenen Ausführungsvariante eines Schaftrohres ohne eingezogenen Kunststoffschlauch, auch die innere Mantelfläche des Schaftrohres mit einer Anti-Haft-Beschichtung versehen sein.

Das biegeweiche Schaftrohr ist in einer bevorzugten Ausführungsform aus Polyetheretherketon (PEEK) geformt und hat vorzugsweise einen Außendurchmesser von weniger als 5 mm und besonders bevorzugt von weniger oder gleich 1,8 mm. Auch die äußere Mantelfläche des Schaftrohres kann mit einer Anti-Haft-Beschichtung versehen sein.

Das normalerweise aus einem Stück gefertigte distale Endstück kann gemäß einer weiteren Ausführungsform zweigeteilt sein und aus einem proximalen und einem distalen Teilstück bestehen, wobei das proximale und das distale Teilstück des Endstücks über Kraft-, Form- oder Stoffschluss miteinander verbunden sind. Vorzugsweise umschließt ein distaler Abschnitt des proximalen Teilstücks den proximalen Abschnitt des distalen Teilstücks, aber auch eine umgekehrte Anordnung der beiden Teilstücke ist gemäß einer weiteren Ausführungsform vorgesehen.

Ist das Endstück zweigeteilt, umschließt die Hülse vorzugsweise beide Teilstücke des distalen Endstücks zumindest auf einer Teillänge dort, wo ein distaler Abschnitt des proximalen Teilstücks einen proximalen Abschnitt des distalen Teilstücks umschließt.

Entsprechende Vertiefungen auf der äußeren Mantelfläche des proximalen Abschnitts des distalen Teilstücks können als Kleberreservoir dienen, mit deren Hilfe sich eine zuverlässige Klebverbindung zwischen dem distalen und dem proximalen Teilstück ergibt.

Bei einer weiteren Ausführungsvariante ist ein distaler Abschnitt des Endstücks als Spitze ausgebildet. Gemäß der Hauptausführungsvariante des Katheters mit einer distalen Öffnung, ist die Spitze entsprechend mit axialer Durchgangsbohrung ausgeführt, die eine distale Mündung des Lumens bildet. Die Spitze besteht bevorzugt aus Polyphenylsulfon (PPSU) oder Polyetheretherketon (PEEK) und kann gegebenenfalls auch mit einer Anti-Haft-Beschichtung versehen sein.

Der maximale Durchmesser der bevorzugt kegelstumpfförmig ausgebildeten Spitze weist vorzugsweise den gleichen Durchmesser auf, wie der Außendurchmesser der distalen Ringelektrode. Die konische Außenfläche der Spitze ist so ausgestaltet, dass sich der Außendurchmesser der Spitze in einem Winkel von 5 bis 85 Grad, besonders bevorzugt von 15 Grad (gemessen zwischen der Steigung der Konusmantelfläche und einer Parallelen zur Katheterlängsachse) zum distalen Ende des Katheters hin verjüngt. In einer besonders bevorzugten Ausführungsvariante erweitert sich die distale Mündungsöffnung der Spitze ab einem Punkt zwischen distalem und proximalem Ende der Spitze zum distalen Katheterende hin. Dies dient u.a. dazu, dass der Führungsdraht leichter eingeführt werden kann. Die sich aus dieser Erweiterung der Mündungsöffnung ergebende Schräge verläuft vorzugsweise in einem Winkel zwischen 20 und 45 Grad gemessen zur Katheterlängsachse.

Je nach Ausführungsform weist das distale Endstück an seinem distalen Ende einen Mündungsdurchmesser von 0,65 bis 2 mm auf. Der kegelstumpfförmig ausgebildete distale Abschnitt des distalen Endstücks dient vor allem dazu, den intraluminalen Vorschub des Katheters zu vereinfachen.

In einer weiteren Ausführungsform kann die Elektrode auch mit einer kopf-, kegel-, trokarförmigen oder sphärischen Stirnfläche ausgebildet sein und so gleichzeitig das distale Endstück bilden.

In einer weiteren Ausführungsvariante weist der Katheter im distalen Bereich wenigstens einen Temperatursensor auf, der die Temperatur der Elektroden, des Katheters, und/ oder des umliegenden Gewebes erfasst und einen entsprechenden Temperaturmesswert einem Messwertaufnehmer zur weiteren Verarbeitung zuführt.

In Hinblick auf die bevorzugten Anwendungen beträgt die Länge des Schaftrohres zwischen 600 und 2000 mm. Ein derartiger Katheter eignet sich für bekannte Anwendungen zur Hochfrequenztherapie von hohlförmigen anatomischen Strukturen, wie Venen oder Gallengängen, ist aber nicht auf diese beschränkt, sondern erschließt darüber hinaus neue Therapieverfahren und Anwendungsgebiete. Für derartige Therapieverfahren kann es sinnvoll sein, zu wissen, wie weit der Katheter bereits in den hohlförmigen anatomischen Strukturen vorangeschoben worden ist. Gemäß einer weiteren Ausführungsvariante kann die äußere Mantelfläche des Schaftrohres dementsprechend mit Abstandsmarkierungen versehen sein.

### Endoluminaler Einsatz des Katheters:

Zuerst wird der Führungsdraht im Lumen des Hohlorgans platziert. Danach wird der Hochfrequenzkatheter über den Führungsdraht geschoben, während der Führungsdraht seine Position beibehält. Wenn die Elektroden des Hochfrequenzkatheters den gewünschten Abschnitt erreicht haben, wird eine hochfrequente Wechselspannung an den Elektroden angelegt. Durch Vorschieben und Zurückziehen bzw. Hin- und Herbewegen können die Position der Elektroden im Hohlorgan verändert werden.

Beim endoluminalen Einsatz wird das zu behandelnde Hohlorgan und eventuell anliegendes Tumorgewebe von Strom durchflossen, erwärmt sich und koaguliert. Behandlungsablauf am Beispiel varikoser Venen:
Ein zu behandelndes Blutgefäß wird zunächst vorzugsweise z.B. in der Nähe des Fußknöchels geöffnet. Anschließend wird ein Führungsdraht unter Umständen mit Hilfe eines Endoskops in die geöffnete Vene eingeführt. Als zweiter Schritt wird der Hochfrequenzkatheter mit seinem distalen Ende voran über den Führungsdraht in die geöffnete Vene eingeführt und bis zum Ende der Vene vorgeschoben. Dabei wird noch keine hochfrequente, zu einer Koagulation führende Wechselspannung, an die Elektrode oder die Elektroden des Hochfrequenzkatheters angelegt.

Nachdem das distale Ende des Hochfrequenzkatheters richtig positioniert ist, kann an die für die Behandlung vorgesehene Elektrode eine hochfrequente Wechselspannung angelegt werden, die ein Schrumpfen der Vene bewirkt. In einer monopolaren Anordnung ist die für die Behandlung vorgesehene Elektrode am distalen Ende des Hochfrequenzkatheters angeordnet. Eine Gegenelektrode wird zuvor als großflächige, neutrale Elektrode am Körper des Patienten angelegt. Falls gemäß einer bevorzugten Variante ein bipolarer Hochfrequenzkatheter verwendet wird, wird die hochfrequente Wechselspannung zwischen einer proximal und einer distal angeordneten Elektrode angelegt.

Um das Blutgefäß auf der gewünschten Länge per Koagulation zu verengen, wird dann der Hochfrequenzkatheter relativ zum Führungsdraht oder zusammen mit dem Führungsdraht langsam in proximaler Richtung zurückgezogen. Die Arbeitsgeschwindigkeit wird dabei an die Geometrie des zu behandelnden Blutgefäßes, sowie an die angelegte hochfrequente Wechselspannung angepasst.

Zur Steigerung des Therapieeffektes kann vor dem Anlegen der hochfrequenten Wechselspannung das sich in der Vene befindende Blut mit einer Manschette über die gesamte Länge der Vene herausgedrückt werden.

Um während des Verfahrens in etwa die Position des Elektrodenkopfes am distalen Ende des Hochfrequenzkatheters abschätzen zu können, ist es vorteilhaft, wenn parallel zu dem Hochfrequenzkatheter vom Verbindungselement der Applikationsvorrichtung ausgehend eine Schnur so gespannt wird, dass sich das Ende der Schnur oder eine markierte Stelle der Schnur außerhalb des Körpers des Patienten etwa gleichauf mit der Kopfelektrode innerhalb des Patienten befindet. Auf diese Weise lässt sich der Hochfrequenzkatheter besonders gefühlvoll und mit gleichmäßiger Geschwindigkeit in proximaler Richtung zurückziehen. Weitere Möglichkeiten der Positionskontrolle sind sonographische Bildgebung, angiographische Bildgebung, Palpation oder Markierungen auf dem Katheterschaft.

Sobald die Elektroden den zu behandelnden Abschnitt eines Blutgefäßes verlassen, werden die Elektroden wieder von der hochfrequenten Wechselspannung getrennt, und der Hochfrequenzkatheter kann ganz aus dem Körper des Patienten zurückgezogen werden.

Falls die Applikationsvorrichtung an ein entsprechendes Steuergerät angeschlossen ist, kann die hochfrequente Wechselspannung während der Koagulation an die jeweiligen Anforderungen adaptiert werden. Falls das Steuergerät so beschaffen ist, dass es beispielsweise ein von der Impedanz zwischen der distalen Elektrode und der Gegenelektrode abhängiges akustisches oder optisches Signal abgibt, kann sowohl die Geschwindigkeit des Zurückziehens des Hochfrequenzkatheters, als auch die Größe der hochfrequenten Wechselspannung besonders leicht den jeweiligen Erfordernissen angepasst werden.

### Behandlungsablauf am Beispiel des Eileiters:

Ein weiteres Einsatzgebiet einer Applikationsvorrichtung mit endoluminaler Ausprägung liegt in der Verengung bzw. Verödung eines Eileiters zu Sterilisationszwecken. Mittels eines Hysteroskops (Endoskop für die Gynäkologie) wird zuerst der Führungsdraht und danach der Hochfrequenzkatheter von der Gebärmutter aus in den zu verschließenden Eileiter eingeführt. Der weitere Verlauf gleicht dem der Verengung von Venen (s.o.): Nach korrekter Positionierung der Elektrode innerhalb des Eileiters wird Hochfrequenzstrom abgegeben und die Elektrode eine definierte Strecke zurückgezogen, so dass sich der koagulierte Bereich bezüglich des Durchmessers zusammenzieht und dadurch verschließt.

### Behandlungsablauf am Beispiel von Tumoren im Bereich des Gallenganges:

Ein weiteres Einsatzgebiet einer Applikationsvorrichtung mit endoskopischer Ausprägung ist die Behandlung obstruierender Tumore im Bereich des Gallenganges (ductus choledochus). Tumore im Bereich des Gallenganges (z. B. das Cholangiokarzinom) haben die Eigenschaft, den Gallengang zusammenzudrücken, wodurch eine teilweise oder vollständige Obstruktion des Gallenganges hervorgerufen wird.

Zur Behandlung dieser Indikation wird das flexible Endoskop (Gastroskop oder Duodenoskop) durch Mundhöhle, Speiseröhre und Magen eingeführt und mit dem distalen Ende vor der großen Papille des Zwölfingerdarms positioniert. Nun wird ein Führungsdraht durch die Papille in den Gallengang eingeführt und über den Abschnitt des Gallenganges, der behandelt werden soll, hinausgeschoben. Anschließend wird der Hochfrequenzkatheter über den Führungsdraht durch den Arbeitskanal des Endoskops vorgeschoben, bis die Elektrodenanordnung des Hochfrequenzkatheters sich in dem zu behandelnden Bereich des Gallenganges befindet. Durch Anlegen einer hochfrequenten Wechselspannung an den Elektroden wird das umliegende Gewebe (z.B. ein Cholangiokarzinom) koaguliert. Je nach Ausdehnung des zu behandelnden Abschnittes wird der Koagulationsvorgang an weiteren Stellen wiederholt. Zur Beseitigung der Obstruktion verbleibt die Elektrode in der jeweiligen Position bis der einzelne Koagulationsvorgang abgeschlossen ist. Dadurch entsteht ein Lumen aus koaguliertem Gewebe mit einem Innendurchmesser, der dem Außendurchmesser der Elektrode entspricht.

Anschließend kann an dieser Stelle, falls erforderlich, ein Stent (Röhrenförmiges Element, mit dem ein Lumen gegen Obstruktion gesichert werden kann) in den Gallengang eingesetzt werden, um eine erneute Obstruktion durch Fibrinabsonderungen zu verhindern.

Die Erfindung wird nachfolgend anhand der Zeichnungen näher erläutert. Es zeigen:
- Figur 1:: einen distalen Endabschnitt eines Katheters zur Hochfrequenztherapie von biologischem Gewebe mit biegeweichem Schaftrohr, einem durchgehenden inneren Lumen, einem in das Lumen des Schaftrohres eingezogenen Kunststoffschlauch, einer Kunststoffspitze, zwei Ringelektroden, zwei elektrischen Leitungen und einem Isolator.
- Figur 1a:: einen Kunststoffschlauch und eine kegelförmige Kunststoffspitze als Bestandteile des Katheters aus Figur 1 in Einzeldarstellung;
- Figur 1b:: ein biegeweiches Schaftrohr als Bestandteil des Katheters aus Figur 1 in Einzeldarstellung;
- Figur 1c:: Elektroden und elektrische Leitungen des Katheters aus Figur 1 in Einzeldarstellung; und
- Figur 1d:: einen zwischen den Elektroden angeordneten Isolator als Bestandteil des Katheters aus Figur 1 in Einzeldarstellung.
- Figur 2:: eine Querschnittsansicht des distalen Endabschnittes eines Katheters gemäß einer zweiten Ausführungsform mit biegeweichem Schaftrohr, einem durchgehenden inneren Lumen, einem zweigeteilten distalen Endstück, zwei Ringelektroden und einem Isolator.
- Figur 3:: eine Querschnittsansicht des distalen Endabschnitts eines Katheters gemäß einer dritten Ausführungsform mit biegeweichem Schaftror, einem innerem Lumen, welches an seinem distalen Ende verschlossen ist, einem zweigeteilten distalen Endstück und einem Kühlsystem.
- Figur 4:: eine perspektivische Ansicht der in Figur 3 dargestellten Ausführungsform des Katheters.

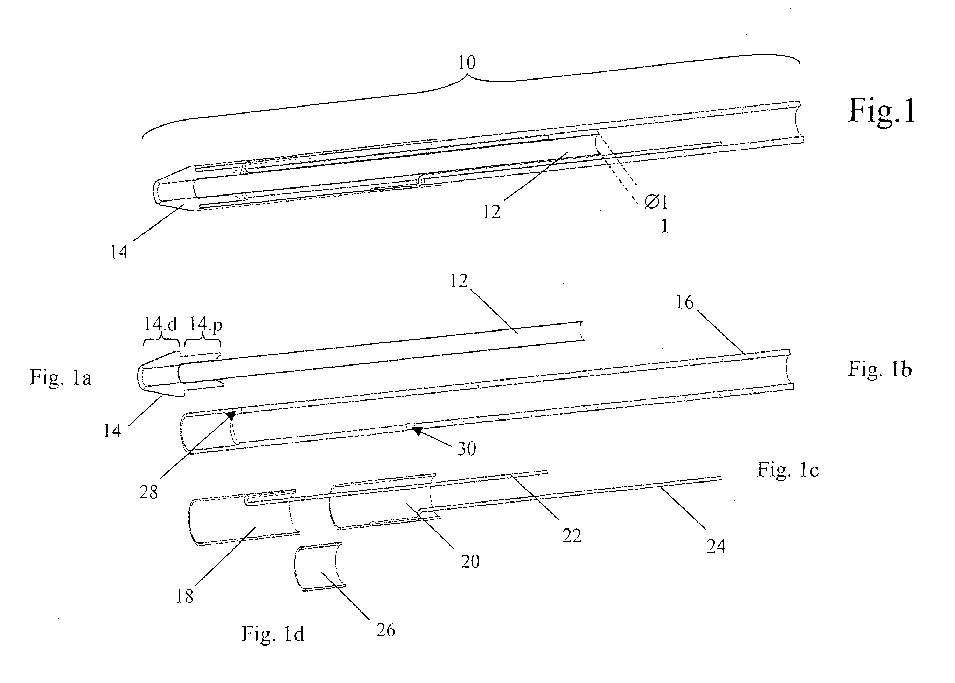

Figur 1 zeigt einen distalen Endabschnitt 10 des erfindungsgemäßen Hochfrequenzkatheters in einer besonders bevorzugten Ausführungsform; mit durchgehendem Lumen 1 zur Durchführung eines Führungsdrahtes; in einer bipolaren Ausführungsvariante.

Das Lumen 1 besitzt einen Durchmesser von 0,81 mm und taugt damit zur Aufnahme eines Führungsdrahtes mit 0,025 Inch Durchmesser. In einer alternativen, nicht dargestellten Ausführungsvariante kann das Lumen 1 auch einen größeren Durchmesser aufweisen, der zur Aufnahme eine Führungsdrahtes mit 0.035 Inch Durchmesser taugt.

Das distale Ende des Katheters weist ein distales Endstück 14 auf. Ein distaler Abschnitt 14.d des Endstücks 14 ist als Kunststoffspitze ausgeblildet, ein zylindrischer, proximaler Abschnitt 14.p des Endstücks 14, der in das distale Ende des Schaftrohres 16 hineinragt, bildet eine Klemmverbindung mit dem distalen Ende des biegeweichen Schaftrohres 16.

Das Endstück 14 weist eine zentrale Bohrung auf, die in das bis zum proximalen Ende des Katheters reichende Lumen 1 mündet. Die äußere Mantelfläche des als kegelstumpfförmige Kunststoffspitze ausgebildeten distalen Abschnitts 14.d des distalen Endstücks 14 verläuft in einem Winkel von 15 Grad gemessen zwischen der konusförmigen Mantelfläche und einer Parallelen der Katheterachse, wobei der Außendurchmesser der Kunststoffspitze stufenlos in den Außendurchmesser einer distalen Ringelektrode 18 übergeht. Am Übergang zwischen distalem 14.d und proximalem Abschnitt 14.p des distalen Endstücks 14 entspricht also der Außendurchmesser des als Kunststoffspitze ausgeformten distalen Abschnitts 14.d dem Außendurchmesser der distalen Ringelektrode 18.

Gemäß der hier dargestellten Ausführungsvariante ist außer der distalen Ringelektrode 18 eine proximale Ringelektrode 20 auf das Schaftrohr 16 des Katheters gezogen. Beide Ringelektroden 18, 20 sind vorzugsweise aus einem biokompatiblen Edelstahl geformt. Die distale Elektrode 18 hat in dieser Ausführungsform die Funktion einer Hülse 18, die die Klemmverbindung zwischen Schaftrohr 16 und proximalem Abschnitt 14.p des Endstücks 14 sichert, indem sie eine radial nach innen gerichtete Klemmkraft auf den von ihr umschlossenen Abschnitt des Schaftrohres 16 ausübt und dadurch die Klemmkraft zwischen Schaftrohr 16 und distalem Endstück 14 erhöht.

In Figur 1 nicht zu sehen ist, dass der proximale Abschnitt 14.p des Endstücks 14 eine Querschnittsveränderung des äußeren Durchmessers z.B. in Form eines ringförmig umlaufenden Vorsprungs aufweisen kann, die eine axiale Verschiebung des Endstücks 14 innerhalb des Schaftrohres 16 erschwert und damit einen noch stärkeren Halt der distalen Elektrode 18 auf dem Schaftrohr 16 garantiert.

Das biegsame Schaftrohr 16 ist bevorzugt aus Polyethteretherketon PEEK geformt.

In axialer Richtung ist zwischen beiden Elektroden 18 und 20 ein Isolator 26 angeordnet, der mit seinen Längsenden unmittelbar an jeweils eine der beiden Elektroden 18 und 20 angrenzt und der wie die Elektroden 18 und 20 ebenfalls außen auf das Schaftrohr 16 des Katheters aufgezogen ist. Der Isolator 26 ist vorzugsweise aus PEEK geformt. Die Elektroden 18 und 20, sowie der Isolator 26 besitzen den gleichen Außendurchmesser von 2,0 mm und vorzugsweise auch einen identischen Innendurchmesser von 1,8 mm.

Die distale Elektrode 18 hat vorzugsweise einen Abstand von 1,8 mm zum distalen Katheterende und eine Länge gemessen in Längsrichtung des Katheters von etwa 5,4 mm. Der Isolator 26 besitzt eine Länge, die etwa 10 % des vorzugsweise 12 mm langen aktiven Teils, bestehend aus distaler Elektrode 18 + Isolator 26 + proximaler Elektrode 20, ausmacht und die damit 1,2 mm beträgt. Die proximale Elektrode 20 besitzt vorzugsweise die gleiche Länge, wie die distale Elektrode 18.

Mit der distalen und proximalen Elektrode 18 und 20 sind zwei elektrische Leitungen 22 und 24 vorzugsweise durch eine Klemmkontaktierung verbunden. Beide Leitungen 22 und 24 bestehen vorzugsweise aus Kupfer und haben jeweils einen Durchmesser von bevorzugt 0,15 mm. Diese sind durch die beiden Durchführungen 28 und 30 des Schaftrohres 16 geführt und verlaufen von der distalen, bzw. proximalen Elektrode 18 bzw. 20 entlang der inneren Mantelfläche des Schaftrohres 16 zum nicht sichtbaren proximalen Ende des Katheters, wo sie mit einem Stecker oder Adapter zum Anschluss an einen Hochfrequenzgenerator verbunden sind.

Die Durchführungen 28 und 30 befinden sich vorzugsweise jeweils auf gegenüberliegenden Seiten des Schaftrohres 16.

Zur Isolierung der Leitungen 28 und 30 und zu deren Fixierung an der Mantelinnenfläche des Schaftrohres 16 ist in das Lumen 1 des Schaftrohres 16 ein Kunststoffschlauch 12 eingezogen, der vom nicht sichtbaren proximalen Ende des Katheters bis an die Übergangsstelle zwischen proximalem 14.p und distalen 14.d Abschnitt des Endstücks 14 verläuft.

Folglich verlaufen die Leitungen 22 und 24 zwischen den Durchführungen 28 und 30 und dem proximalen Ende des Katheters in einem Zwischenraum zwischen Schaftrohr 16 und einem inneren Kunststoffschlauch 12.

Der Durchmesser des Katheterlumens 1 entspricht in dieser Ausführungsform dem Innendurchmesser 1 des Kunststoffschlauchs.
Figuren 1a bis 1d zeigen den Katheter gemäß Figur 1 in Einzeldarstellungen. In Figur 1 a ist der Kunststoffschlauch 12 mit dem als Kunststoffspitze ausgeführten distalen Abschnitt 14.d des Endstücks 14 gezeigt. Am distalen Ende der Kunststoffspitze ist erkennbar, dass der Eingang zur zentralen Bohrung trichterförmig ausgebildet ist, wobei die innere Mantelfläche an dieser Stelle mit einem Winkel von etwa 45 Grad, gemessen zwischen der konusförmigen Mantelinnenfläche und einer Parallelen zur Katheterachse, verläuft. Durch diese trichterförmige Ausbildung wird die Aufnahme eines Führungsdrahtes erleichtert.

Der Kunststoffschlauch 12 ist bis zur Übergangsstelle zwischen proximalem 14.p und distalen 14.d Abschnitt des Endstücks 14 in der zentralen Bohrung des Endstücks 14 vorgeschoben. Der Kunststoffschlauch 12 und das Endstück 14 können bei dieser Ausführungsvariante über eine Presspassung, oder auch durch eine Schweiß oder Klebeverbindung miteinander verbunden sein.

Firgur 1 b zeigt eine Schnittdarstellung des biegsamen Schaftrohres 16. Gut zu erkennen sind hierin die beiden Durchführungen 28 und 30 für die Leitungen 22 und 24.
Figur 1c zeigt eine Schnittdarstellung der distalen 18 und der proximalen Elektrode 20 mit den zugehörigen elektrischen Leitungen 22 und 24. Die elektrischen Leitungen 22 und 24 verlaufen vom proximalen Ende des Katheters bis zu den für sie jeweils vorgesehenen Durchführungen 28 und 30 parallel zu der Katheterlängsachse. Nach Ihrem Durchtritt durch die jeweilige Durchführung in dem Mantel des Schaftrohres 16, wofür sie einen ersten Knick um 90 Grad entsprechen der radialen Ausrichtung der Durchführungen 28 und 30 aufweisen, verlaufen sie auch nach ihrem Durchtritt durch die Mantelfläche des Schaftrohres 16 vorzugsweise in axial - distaler oder axial - proximaler Richtung, und weisen entsprechend einen zweiten Knick um 90 Grad auf. Durch das Aufziehen der Ringelektroden 18 und 20 auf das Schaftrohr 16 werden die Leitungen 22 und 24 durch die radial nach innen wirkende Klemmkraft der Elektroden 18 und 20 zwischen der jeweiligen Elektrode 18 bzw. 20 und dem Schaftrohr 16 eingeklemmt, wodurch die Klemmkontaktierung zwischen den Leitungen 22 und 24 und den Elektroden 18 und 20 zustande kommt. In alternativen Ausgestaltungsvarianten kann die Verbindung zwischen den Leitungen und der jeweiligen Elektrode zusätzlich oder ausschließlich durch einen Stoffschluss, wie z.B. Kleben oder Löten hergestellt sein.

Figur 1d zeigt eine Schnittdarstellung des Isolators 26.

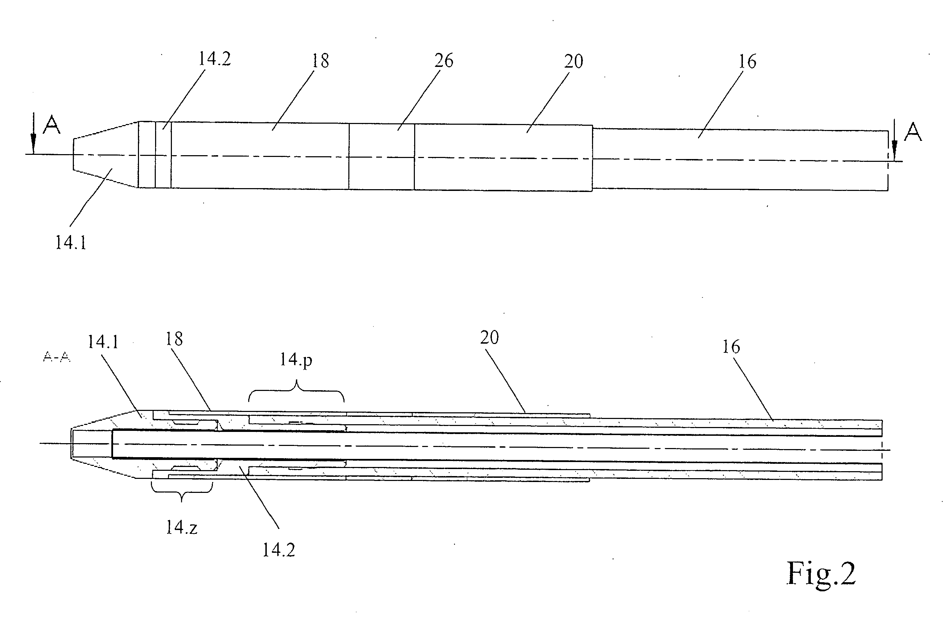

Figur 2 zeigt einen distalen Abschnitt 10 des erfindungsgemäßen Hochfrequenzkatheters in einer weiteren Ausführungsvariante, mit durchgehendem Lumen 1 zur Durchführung eines Führungsdrahtes; in einer bipolaren Ausführungsvariante.

Der Unterschied zu der in Figur 1 dargestellten Ausführungsvariante liegt vor allem darin, dass das distale Endstück 14 zweigeteilt ist und aus einem distalen 14.1 und einem proximalen Teilstück 14.2 besteht.

Ein proximaler zylindrischer Abschnitt 14.p des proximalen Teilstücks 14.2 ragt dabei in das distale Ende des Schaftrohres 16 hinein und bildet mit dem distalen Ende des biegeweichen Schaftrohres 16 eine Klemmverbindung. Wie in Figur 1 nicht zu sehen war, ist in dieser Abbildung eine Querschnittsveränderung auf der äußeren Mantelfläche des proximalen Abschnittes 14.p des proximalen Teilstücks 14.2 zu erkennen. Wie schon beschrieben verbessert diese Querschnittsveränderung die Belastbarkeit der Klemmverbindung und erschwert eine axiale Bewegung des proximalen Teilstücks 14.2.

Das proximale Teilstück 14.2 ist vorzugsweise aus Metall gefertigt, um eine größere radiale Krafteinwirkung durch die Hülse bzw. distale Ringelektrode 18 aufnehmen zu können und die Belastbarkeit der Klemmverbindung zu erhöhen.

An der Stelle, an der das proximale Teilstück 14.2 aus dem Schaftrohr 16 herausragt, erweitert sich der Außendurchmesser des Teilstücks 14.2 auf den Außendurchmesser des Schaftrohres 16 und fixiert das Teilstück 14.2 bezüglich einer distal gerichteten axialen Bewegung.

Eine weitere Querschnittserweiterung des äußeren Durchmessers des proximalen Teilstücks 14.2 auf den Außendurchmesser der distalen Ringelektrode 18 bildet einen Flansch am distalen Ende des proximalen Teilstücks 14.2 und verhindert eine axiale Verschiebung der distalen Ringelektrode 18 in distaler Richtung.

Um das distale Teilstück 14.1 mit dem proximalen Teilstück 14.2 zu verbinden umschließt ein distaler Abschnitt 14.z des proximalen Teilstücks 14.2 den proximalen Abschnitt 14.z des distalen Teilstücks 14.1. Auf der äußeren Mantelfläche des distalen Teilstücks 14.1 sind in einem proximalen Abschnitt 14.z Querschnittsveränderungen vorgesehen, die zu den Querschnittsveränderungen der inneren Mantelfläche des proximalen Teilstücks 14.2 in einem distalem Abschnitt 14.z kompatibel sind und so eine Einrast-Verbindung zwischen den beiden Teilstücken 14.1 und 14.2 ermöglichen. Für eine Sicherung der Klemm-, bzw. Einrast-Verbindung sorgt auch in dieser Ausführungsvariante die distale Ringelektrode 18, die durch eine radiale Krafteinwirkung auf die Abschnitte 14.z und 14.p beide Verbindungen sichert.

Das distale Teilstück 14.1 weist auch in dieser Ausführungsvariante einen als Spitze ausgeformten distalen Abschnitt auf und ist vorzugsweise aus Kunststoff gefertigt. Die in Figur 2 beschriebene Variante des zweigeteilten distalen Endstücks 14 weist gegenüber der in Figur 1 beschriebenen Ausführungsvariante den Vorteil auf, dass die beiden Teilstücke 14.1 und 14.2 des distalen Endstücks 14 aus unterschiedlichen Materialien geformt sein können.

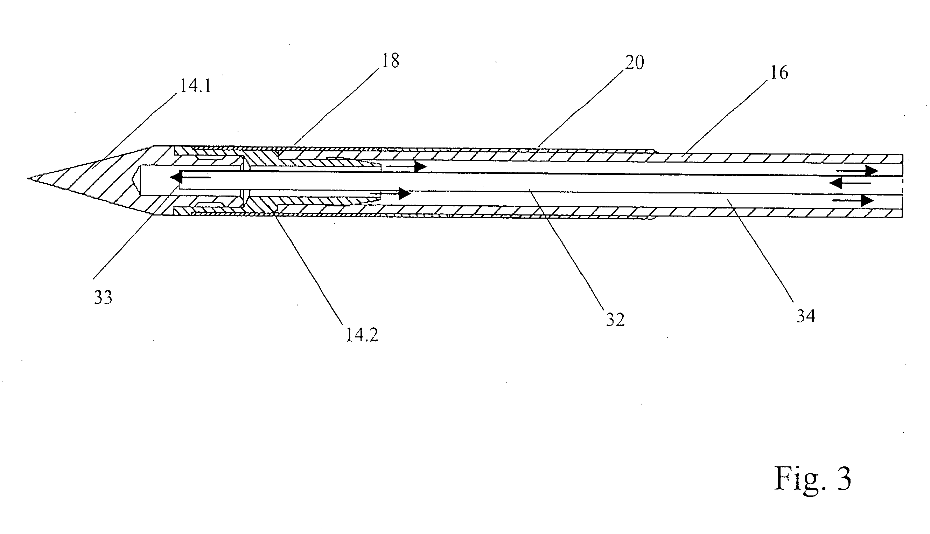

Figur 3 zeigt das distale Ende einer dritten Ausführungsform des Katheters in einer Querschnitts-Darstellung.

Wie bei allen Ausführungsformen des Katheters ist auch gemäß dieser Ausführungsform ein biegeweiches Schaftror 16 vorgesehen, welches ein Lumen 1 umschließt. Anders, als bei den ersten beiden Ausführungsformen, ist das Lumen 1 des hier dargestellten Katheters am distalen Ende verschlossen.

Wie auch bei der zweiten Ausführungsform geht auch bei der hier dargestellten Ausführungsform der Durchmesser des Lumens 1 vom Innendurchmesser des biegeweichen Schaftrohres 16 in den Innendurchmesser eines zweigeteilten distalen Endstücks 14.1, 14.2 über. Der proximale Teil des distalen Endstücks 14.2 weist dabei eine Durchgangsbohrung auf. Im Unterschied zu den bislang dargestellten Ausführungsformen weist der distale Teil 14.1 des zweigeteilten Endstücks jedoch eine Sackbohrung auf. Dementsprechend endet das Lumen des Katheters, im Gegensatz zu den Ausführungsformen 1 und 2, in der Sackbohrung des distalen Teils 14.1 des zweigeteilten Endstücks. Der Innenraum des Katheters ist somit umschlossen, sodass keine Flüssigkeit aus dem Inneren des Katheters nach außen treten kann oder umgekehrt.

Der in Figur 3 dargestellte Katheter verfügt darüber hinaus über ein Kühlsystem mit einem hier nicht dargestellten proximalen Zu- und Abfluss für ein Kühlmedium. Dabei ist das hier dargestellte Kühlsystem durch ein Zuleitungsrohr 32 gebildet, welches lediglich am nicht dargestellten proximalen Ende des Katheters gehalten ist und dessen distales Ende sich relativ frei im distalen Ende des Katheters und wie hier dargestellt in der Bohrung des distalen Endstücks 14.1, 14.2 bewegen kann. Das Zuleitungsrohr 32 ist wie das Schaftrohr 16 aus einem biegeweichen Kunststoff, z.B. PEEK, hergestellt. Dabei kann ein Katheter gemäß dieser Ausführungsform mit einer Kühlflüssigkeit gespült werden. Hierfür wird dem Zuleitungsrohr 32 über einen proximalen Zufluss ein Kühlmedium zugeführt, welches aus einer distalen Mündung 33 des Zuleitungsrohres 32 austritt und dabei vor allem das distale Ende des Katheters kühlt. Aufgrund des verschlossenen distalen Schaftendes strömt das Kühlmedium daraufhin durch einen Zwischenraum 34 zwischen dem Außendurchmesser des Zuleitungsrohres 32 und dem Innendurchmesser des biegeweichen Schaftrohres 16 zum proximalen Ende des Schaftes zurück, wo es an dem proximalen Abfluss aus dem Katheter austritt.

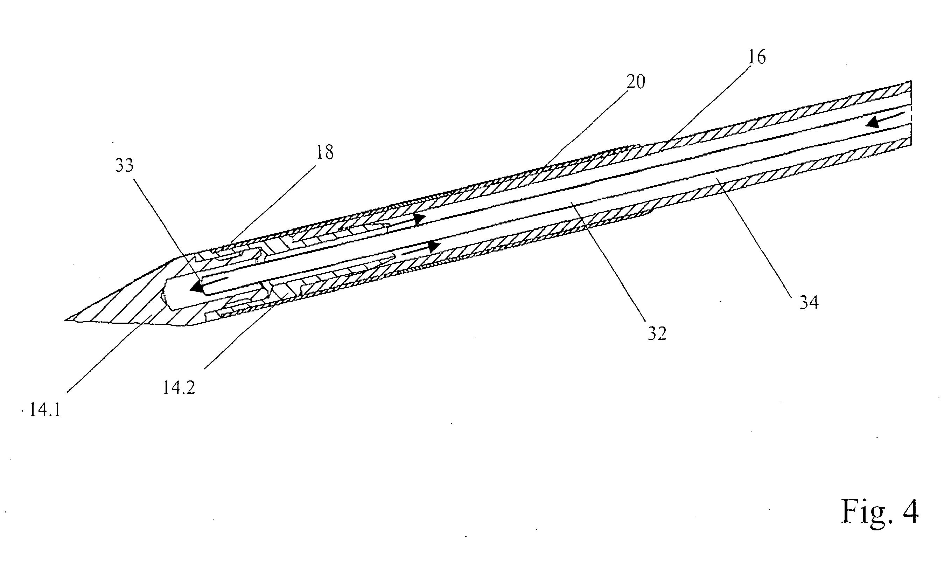

Figur 4 ist eine perspektivische Ansicht der in Figur 3 dargestellten Ausführungsform des Katheters. Die Beschreibung der Figur 3 kann somit auf die Figur 4 vollständig übertragen werden.

## Patentansprüche

1. Katheter für die Behandlung von Körperlumen mit
- einem biegeweichen Schaftrohr (16) aus biokompatiblem Kunststoff, der ein Lumen (1) umschließt,
- einem distalen Abschnitt (10) des biegeweichen Schaftrohres (16) mit wenigstens einer Elektrode (18, 20) zur Abgabe hochfrequenten Wechselstroms an das den Katheter im Behandlungsfall umgebende Körpergewebe,
- einer mit der Elektrode (18, 20) elektrisch leitend verbundenen elektrischen Leitungen (22, 24), die bis zum proximalen Ende des Katheters reicht,
- einem am distalen Ende des biegeweichen Schaftrohrs (16) befestigten distalen Endstück (14), wobei
ein proximales Ende des distalen Endstücks (14.p) in das Lumen des biegeweichen Schaftrohres (16) hineinragt und dort eine Klemmverbindung mit dem distalen Ende des biegeweichen Schaftrohres (16) bildet, die durch eine Hülse (18) in Form eines Hohlzylinders dadurch gesichert ist, dass die Hülse (18) eine radial wirkende Klemmkraft auf die Klemmverbindung ausübt,
**dadurch gekennzeichnet, dass**
die Hülse (18) elektrisch leitend ist und die Elektrode (18) bildet.

2. Katheter nach Anspruch 1, **dadurch gekennzeichnet, dass** das distale Endstück (14) elektrisch leitend ist und eine distale Elektrode bildet.

3. Katheter nach einem der Ansprüche 1 bis 2, **dadurch gekennzeichnet, dass** das distale Endstück (14) mindestens eine Querschnittsveränderung des Außendurchmessers aufweist.

4. Katheter nach einem der Ansprüche 1 bis 3, **dadurch gekennzeichnet, dass** die Hülse (18) mindestens eine Querschnittsveränderung des Innendurchmessers aufweist.

5. Katheter nach einem der Ansprüche 1 bis 4, **dadurch gekennzeichnet, dass** das distale Endstück (14) zweigeteilt ist und aus einem proximalen (14.2) und einem distalen (14.1) Teilstück besteht, wobei das proximale Ende (14.z) des distalen Teilstücks (14.1) über eine Teillänge (14.z) in das distale Ende (14.z) des proximalen Teilstücks (14.2) hineinragt oder umgekehrt und über diese Teillänge (14.z) eines der beiden Teilstücke (14.1) bzw. (14.2) durch Kraft-, Form- oder Stoffschluss mit dem jeweils anderen Teilstück verbunden ist.

6. Katheter nach Anspruch 5, **dadurch gekennzeichnet, dass** die Hülse (18) beide Teile des distalen Endstücks (14) zumindest auf einer Teillänge dort umschließt, wo ein distaler Abschnitt (14.z) des proximalen Teilstücks (14.2) einen proximalen Abschnitt (14.z) des distalen Teilstücks (14.1) umschließt.

7. Katheter nach einem der Ansprüche 1 bis 6, **dadurch gekennzeichnet, dass** in das Lumen (1) des biegeweichen Schaftrohres (16) ein Kunststoffschlauch eingezogen ist, wodurch sich der Durchmesser des Katheterlumens (1) auf den Innendurchmesser des Kunststoffschlauches reduziert, wobei das biegeweiche Schaftrohr (16) mit dem Kunststoffschlauch (12) durch Schweißen, Kleben oder durch eine Pressverbindung flüssigkeitsdicht verbunden ist.

8. Katheter nach einem der Ansprüche 1 bis 7, **dadurch gekennzeichnet, dass** das biegeweiche Schaftrohr (16) mindestens zwei Durchführungen (28, 30) zur Durchführung von elektrischen Leitungen (22, 24) besitzt.

9. Katheter nach einem der Ansprüche 1 bis 8, **dadurch gekennzeichnet, dass** auf das biegeweiche Schaftrohr mindestens 2 Ringelektroden (18, 20) aufgezogen sind.

10. Katheter nach einem der Ansprüche 1 bis 9, **dadurch gekennzeichnet, dass** das distale Endstück (14) mit einer zentralen, axialen Durchgangsbohrung ausgeführt ist, die einen distalen Endabschnitt des Lumens (1), sowie dessen distale Mündung bildet.

11. Katheter nach einem der Ansprüche 1 bis 10, **dadurch gekennzeichnet, dass** das Lumen (1) des Katheters vom proximalen zum distalen Ende durchgehend ist und über eine proximale und eine distale Mündung verfügt, wobei der Katheter dazu ausgebildet ist, dass ein Führungsdraht über die proximale Mündung eingeführt werden kann, dass er durch das durchgehende Lumen zum distalen Ende vorangeschoben werden kann und dass er durch die distale Mündung wieder austreten kann.

12. Katheter nach einem der Ansprüche 1 bis 11, **dadurch gekennzeichnet, dass** der Katheter am distalen Ende verschlossen ist.

13. Katheter nach Anspruch 12, **dadurch gekennzeichnet, dass** der Katheter über ein Kühlsystem, mit einem proximalen Zu- und Abfluss für ein Kühlmedium, verfügt.

## Claims

1. Catheter for treating body lumina having
- a flexible shaft tube (16) which is of biocompatible plastics material and which surrounds a lumen (1),
- a distal portion (10) of the flexible shaft tube (16) having at least one electrode (18, 20) for transmitting high-frequency alternating current to the body tissue which surrounds the catheter in the case of treatment,
- an electrical line (22, 24) which is connected to the electrode (18, 20) in an electrically conductive manner and which extends as far as the proximal end of the catheter,
- a distal end piece (14) which is fixed to the distal end of the flexible shaft tube (16), wherein
a proximal end of the distal end piece (14.p) protrudes into the lumen of the flexible shaft tube (16) and forms at that location a clamping connection with the distal end of the flexible shaft tube (16), which connection is secured by a sleeve (18) in the form of a hollow cylinder in that the sleeve (18) applies a radially acting clamping force to the clamping connection,
**characterised in that**
the sleeve (18) is electrically conductive and forms the electrode (18).

2. Catheter according to claim 1, **characterised in that** the distal end piece (14) is electrically conductive and forms a distal electrode (18).

3. Catheter according to either claim 1 or claim 2, **characterised in that** the distal end piece (14) has at least one cross-sectional variation of the outer diameter.

4. Catheter according to any one of claims 1 to 3, **characterised in that** the sleeve (18) has at least one cross-sectional variation of the inner diameter.

5. Catheter according to any one of claims 1 to 4, **characterised in that** the distal end piece (14) is divided into two and comprises a proximal part-piece (14.2) and a distal part-piece (14.1), wherein the proximal end (14.z) of the distal part-piece (14.1) projects via a part-length (14.z) into the distal end (14.z) of the proximal part-piece (14.2), or vice versa, and is connected to the other part-piece by means of this part-length (14.z) of one of the two part-pieces (14.1) or (14.2) by non-positive-locking, positive-locking or material engagement, respectively.

6. Catheter according to claim 5, **characterised in that** the sleeve (18) surrounds both portions of the distal end piece (14) at least over a part-length at locations where a distal portion (14.z) of the proximal part-piece (14.2) surrounds a proximal portion (14.z) of the distal part-piece (14.1).

7. Catheter according to any one of claims 1 to 6, **characterised in that** a plastics hose is drawn into the lumen (1) of the flexible shaft tube (16), whereby the diameter of the catheter lumen (1) is reduced to the inner diameter of the plastics hose, wherein the flexible shaft tube (16) is connected to the plastics hose (12) by welding, adhesive-bonding or by a press-fit connection in a fluid-tight manner.

8. Catheter according to any one of claims 1 to 7, **characterised in that** the flexible shaft tube (16) has at least two conduits (28, 30) for the introduction of electrical lines (22, 24).

9. Catheter according to any one of claims 1 to 8, **characterised in that** at least two ring electrodes (18, 20) are drawn onto the flexible shaft tube.

10. Catheter according to any one of claims 1 to 9, **characterised in that** the distal end piece (14) is constructed to have a central, axial through-hole which forms a distal end portion of the lumen (1) and the distal opening thereof.

11. Catheter according to any one of claims 1 to 10,**characterised in that** the lumen (1) of the catheter is continuous from the proximal end to the distal end and has a proximal opening and a distal opening, wherein the catheter is constructed in such a manner that a guide wire can be introduced via the proximal opening, it can be advanced through the continuous lumen to the distal end and it can be discharged by the distal opening again.

12. Catheter according to any one of claims 1 to 11, **characterised in that** the catheter is closed at the distal end.

13. Catheter according to claim 12, **characterised in that** the catheter has a cooling system having a proximal supply and discharge for a cooling medium.

## Revendications

1. Cathéter pour le traitement de lumen corporel avec :
- un tube de tige (16) souple en flexion en matière plastique biocompatible cernant un lumen (1) ;
- une section distale (10) du tube de tige (16) souple en flexion avec au moins une électrode (18, 20) pour émettre un courant alternatif à haute fréquence au niveau du tissu de corps entourant le cathéter en cas de traitement ;
- une conduite électrique (22, 24) reliée de façon électriquement conductrice à l'électrode (18, 20) allant jusqu'à l'extrémité proximale du cathéter ;
- une pièce d'extrémité (14) distale fixée à l'extrémité distale du tube de tige (16) souple en flexion ;
une extrémité proximale de la pièce d'extrémité (14.p) distale rentrant dans le lumen du tube de tige (16) souple en flexion et formant à cet endroit une liaison par serrage avec l'extrémité distale du tube de tige (16) souple en flexion ainsi sécurisée par une douille (18) prenant la forme d'un cylindre creux que la douille (18) exerce une force de serrage agissant dans le plan radial sur la liaison par serrage, **caractérisé en ce que** la douille (18) est électriquement conductrice et forme l'électrode (18).

2. Cathéter selon la revendication 1, **caractérisé en ce que** la pièce d'extrémité (14) distale est électriquement conductrice et forme une électrode distale.

3. Cathéter selon l'une quelconque des revendications 1 à 2, **caractérisé en ce que** la pièce d'extrémité (14) distale comporte au moins une variation de section transversale du diamètre extérieur.

4. Cathéter selon l'une quelconque des revendications 1 à 3, **caractérisé en ce que** la douille (18) comporte au moins une variation de section transversale du diamètre intérieur.

5. Cathéter selon l'une quelconque des revendications 1 à 4, **caractérisé en ce que** la pièce d'extrémité (14) distale est divisée en deux et se compose d'une pièce proximale (14.2) et d'une pièce distale (14.1), l'extrémité proximale (14.z) de la pièce distale (14.1) rentrant, au-delà d'une longueur partielle (14.z), dans l'extrémité distale (14.z) de la pièce proximale (14.2) ou inversement et étant reliée, au-delà de cette longueur partielle (14.z) d'une des deux pièces (14.1) et/ou (14.2), à l'autre pièce respectivement, par complémentarité de forces, de formes ou de matières.

6. Cathéter selon la revendication 5, **caractérisé en ce que** la douille (18) enceint deux parties de la pièce d'extrémité (14) distale au moins sur une longueur partielle, là où une section distale (14.z) de la pièce proximale (14.2) enceint une section proximale (14.z) de la pièce distale (14.1).

7. Cathéter selon l'une quelconque des revendications 1 à 6, **caractérisé en ce qu'**un tuyau flexible en matière plastique est inséré dans le lumen (1) du tube de tige (16) souple en flexion, réduisant ainsi le diamètre du lumen de cathéter (1) au diamètre intérieur du tuyau flexible en matière plastique, le tube de tige (16) souple en flexion étant relié, de façon étanche aux fluides, au tuyau flexible en matière plastique (12) par soudure, collage, ou par le biais d'une liaison compressée.

8. Cathéter selon l'une quelconque des revendications 1 à 7, **caractérisé en ce que** le tube de tige (16) souple en flexion possède au moins deux passages (28, 30) pour le passage de câbles électriques (22, 24).

9. Cathéter selon l'une quelconque des revendications 1 à 8, **caractérisé en ce qu'**au moins 2 électrodes annulaires (18, 20) sont tirées sur le tube de tige souple en flexion.

10. Cathéter selon l'une quelconque des revendications 1 à 9, **caractérisé en ce que** la pièce d'extrémité (14) distale est réalisée avec un alésage traversant axial central formant une section distale d'extrémité du lumen (1), ainsi que son embouchure distale.

11. Cathéter selon l'une quelconque des revendications 1 à 10, **caractérisé en ce que** le lumen (1) du cathéter est traversant d'une extrémité proximale à l'extrémité distale et dispose d'une embouchure proximale et d'une embouchure distale, le cathéter étant réalisé pour pouvoir introduire un guide-fil via l'embouchure proximale, que ledit fil peut être avancé à travers ledit lumen traversant en direction de l'extrémité distale et pour le sortir de nouveau à travers l'embouchure distale.

12. Cathéter selon l'une quelconque des revendications 1 à 11, **caractérisé en ce que** le cathéter est fermé au niveau de l'extrémité distale.

13. Cathéter selon la revendication 12, **caractérisé en ce que** le cathéter dispose d'un système de refroidissement, avec un débit d'alimentation et de refoulement proximal pour un agent de refroidissement.
